# EUROPEAN PATENT APPLICATION

(11) **EP 4 434 456 A1**
(43) Date of publication of application: **25.09.2024**
(21) Application number: 24163301.5
(22) Date of filing: 13.03.2024
(51) Int. Cl.: A61B 5/11, A61B 5/16, A61B 5/024, A61B 5/08

(54) **NECKBAND**

(30) Priority: 21.03.2023 EP 23163210
(71) Applicant: Sony Group Corporation, Tokyo 108-0075 (JP)
(72) Inventor: MISEIKIS, Justinas, 70327 Stuttgart (DE)
(74) Representative: MFG Patentanwälte Meyer-Wildhagen Meggle-Freund Gerhard PartG mbB

(57) **Abstract**

The disclosure pertains to a neckband to be worn on a shoulder of a user, wherein the neckband includes a sensor that is configured to acquire sensor data; and a processor that is configured to determine, based on the sensor data, a state of a user who is wearing the neckband on his shoulder; and perform an action according to the determined state.

## Description

### TECHNICAL FIELD

The present disclosure pertains to a neckband.

### TECHNICAL BACKGROUND

Audio playback devices are generally known. For example, an audio playback device may be coupled with a user terminal (e.g., a smartphone or a notebook) via Bluetooth and may playback audio received from the user terminal. The audio playback device may be configured as a neckband for being worn on a shoulder of a user.

Although there exist audio playback devices configured as a neckband, it is generally desirable to provide an improved neckband.

### SUMMARY

According to a first aspect, the disclosure provides a neckband to be worn on a shoulder of a user, the neckband comprising: a sensor configured to acquire sensor data; and a processor configured to: determine, based on the sensor data, a state of a user who is wearing the neckband on his shoulder; and perform an action according to the determined state.

Further aspects are set forth in the dependent claims, the drawings and the following description.

### BRIEF DESCRIPTION OF THE DRAWINGS

Embodiments are explained by way of example with respect to the accompanying drawings, in which:
Fig. 1 illustrates a neckband according to an embodiment;
Fig. 2 illustrates a hardware configuration of a neckband according to an embodiment;
Fig. 3 illustrates a method according to an embodiment; and
Fig. 4 illustrates an embodiment of a general-purpose computer.

### DETAILED DESCRIPTION OF EMBODIMENTS

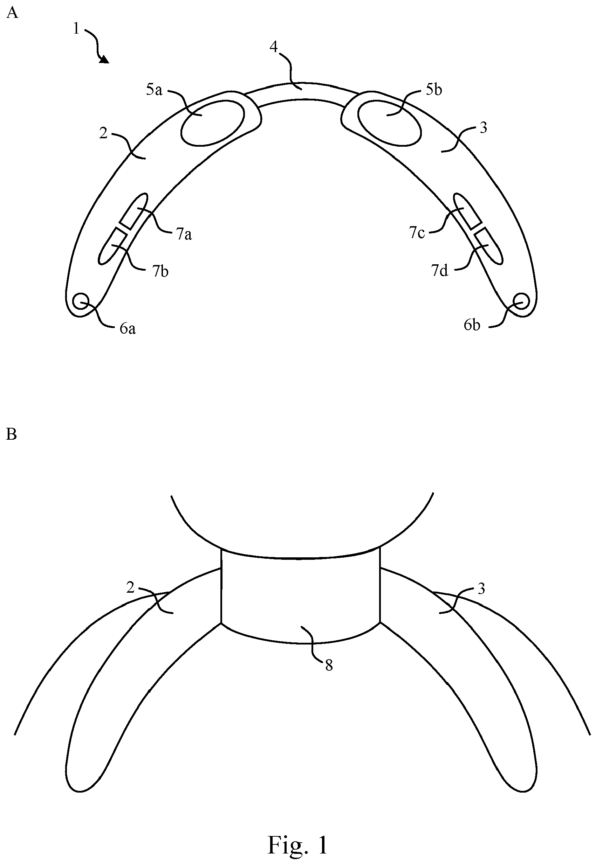

Before a detailed description of the embodiments under reference of Fig. 1 is given, general explanations are made.

As mentioned in the outset, audio playback devices are generally known. For example, an audio playback device may be coupled with a user terminal (e.g., a smartphone or a notebook) via Bluetooth and may playback audio received from the user terminal. The audio playback device may be configured as a neckband for being worn on a shoulder of a user.

Furthermore, assisted living, ageing population and people with disability living independently at home is an emerging issue in some instances where technology may enable people to live significantly longer independently at home instead of having to go to care home facilities.

Multiple sports or specifically designed devices such as armbands are used for care applications in some instances. However, such devices may be not convenient to wear and/or to charge, and may be obtrusive if people are not used to wearing them. Also, such devices may often be used just for observation and may not be helpful for the wearer on a daily basis, but instead may cause objections to use them.

Accordingly, some embodiments of the disclosure pertain to a neckband to be worn on a shoulder of a user, wherein the neckband includes: a sensor that is configured to acquire sensor data; and a processor that is configured to: determine, based on the sensor data, a state of a user who is wearing the neckband on his shoulder; and perform an action according to the determined state.

The neckband may include a right portion, a left portion and a middle portion. The user may wear the right portion on his right shoulder and the left portion on his left shoulder. The middle portion may connect the right portion with the left portion and may be provided behind a neck of the user. Due to its form, the neckband may be easy to put on and take off. The middle portion may be flexible for even easier putting on and/or putting down the neckband. The neckband may be worn on the shoulders, which may be not obtrusive and may be convenient for all-day use without tiring or disturbing the user who is wearing the neckband. Customizable skins may be created to make it more appealing for the wearer.

The sensor may include any device that is capable of sensing a quantity based on which the processor may determine the state of the user. Examples of sensors (and of corresponding quantities sensed by the sensor) are given below. The sensor data may represent a result of the sensing by the sensor. For example, the sensor may acquire the sensor data at predefined times (e.g., every 10 milliseconds, every second, every minute) and/or upon request by the processor. The sensor may provide the sensor data to the processor via a suitable protocol, e.g., via Universal Serial Bus (USB), Peripheral Component Interconnect (PCI), PCI-Extended (PCI-X), PCI Express (PCI-E), parallel port (IEEE 1284), serial port (RS-232), Camera Serial Interface (CSI), Serial Peripheral Interface (SPI) or the like.

Since the sensor is included in the neckband, the sensor may be able to sense a quantity at a position of the user who is wearing the neckband and/or to sense a biological quantity (e.g., a vital sign) of the user who is wearing the neckband.

The processor may include any circuitry that is capable of processing information, e.g., of determining a state of the user based on the sensor data and of performing the action according to the determined state. For example, the processor may include a programmed microprocessor, a field-programmable gate array (FPGA), an application-specific integrated circuit (ASIC) or the like. The processor may be configured to execute software instructions.

The neckband may further include a storage unit. The storage unit may include a non-volatile portion that stores the software instructions for the processor. The non-volatile portion may be based on, e.g., flash memory, electrically erasable programmable read-only memory (EEPROM), magnetic memory or the like. The storage unit may further include a volatile portion that stores temporary data generated at runtime by the processor. The volatile portion may be based on dynamic random-access memory (DRAM), extended data output random-access memory (EDO-RAM), fast page mode random-access memory (FPM-RAM) or the like.

The neckband may include a communication unit for exchanging data with another device (e.g., a smartphone, notebook, television set, hearing aid or the like) of the user and/or for connecting to a communication network such as the internet or an intranet. The communication unit may include a wired interface such as USB, Ethernet, parallel port, serial port or the like. The communication unit may include a wireless interface such as Wi-Fi (IEEE 802.11), Bluetooth, ZigBee, ultra-wide band (UWB), New Radio (NR), Long Term Evolution (LTE), High Speed Packet Access (HSPA) or the like.

The neckband may further include a battery that stores electrical energy. The battery may provide the stored electrical energy to the processor, to the sensor, to the storage unit and/or to the communication unit. The battery may be rechargeable. The recharging of the battery may be performed wired via USB (e.g., USB-C, Micro-USB or the like), lightning, a coaxial power connector, a jack plug, a cold-device plug or the like, and/or wireless via Qi or the like. Due to the size of the neckband, the battery may be large enough for a multi-day battery life (e.g., a capacity for storing electrical energy that lasts for several days). The neckband may be charged on a (e.g., convenient) stand. The stand may be provided at a bedside of the user such that the user may have the neckband in reach even when the neckband is charging (e.g., at night when the user is sleeping).

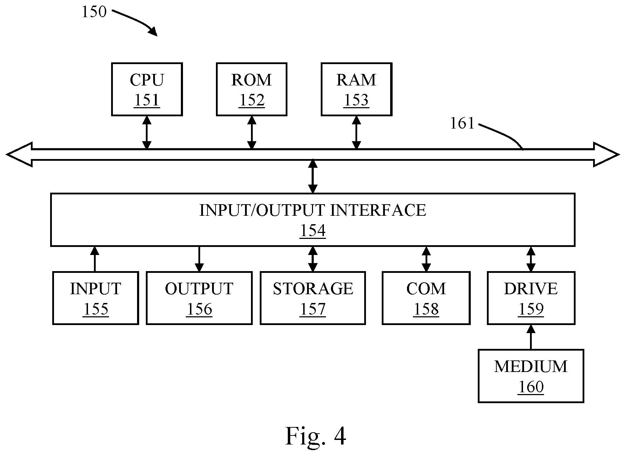

The neckband may include a general-purpose computer as described with reference to Fig. 4.

The determining of the state of the user may be based on determining whether the sensor data fulfills a predefined condition (e.g., whether the sensor data exceeds a threshold). The determining of the state of the user may include performing a classification with an artificial neural network based on the sensor data.

The action may be predefined and may include transmitting information via the communication unit. For example, the action may provide assistance to the user who is wearing the neckband and/or may monitor the user. For example, the neckband may provide information for observing the state of the user and provide such information to a family member, caretaker and/or doctor who is given approval to receive this information. For example, the processor may daily transmit a log of the determined state of the user from a previous day to a predefined contact.

The neckband may have a similar design to Sony SRS-NB 10 or SRS-NS7, and may be configured as a personal care device which not only observes the state of the person, but also provides audio-based assistance to the wearer. At the same time, the neckband may provide immersive and/or useful audio experience to the user who is wearing the neckband and may, thus, enhance everyday activities of the user.

In some embodiments, the action includes notifying a predefined contact of the determined state.

The processor may notify the predefined contact via a communication channel such as a telephone call, a short message service (SMS) message, an e-mail, a chat message on an instant messaging service, a paging signal or the like. The processor may have several communication channels configured.

The notifying may include automatically generating and sending a message (e.g., a predefined message and/or a message generated by a chat bot such as ChatGPT). The notifying may include establishing a communication session with the predefined contact and prompting the user to generate a message (e.g., by speaking and/or typing) for the predefined contact.

The predefined contact may include a relative (e.g., a spouse or a child) of the user, a neighbor of the user, a doctor, a nurse, an emergency medical service (EMS) or the like. The storage unit may store several predefined contacts.

The processor may select, based on the determined state, which communication channel should be used for the notification, which predefined contact(s) should be notified and/or whether a message for the predefined contact should be generated automatically or generated by the user. For example, the processor may estimate, based on the sensor data and on a predefined criterion, whether the determined state corresponds to a (medical) emergency that requires a quick response, and may automatically transmit corresponding information to a predefined contact that is configured as an emergency contact, e.g., a doctor, a nurse and/or an EMS.

In some embodiments, the sensor includes an inertial measurement unit (IMU); and the determining of the state of the user includes determining a movement of the user based on the IMU.

For example, the IMU may detect an acceleration and/or a rotation of the neckband (and, thus, of the user who is wearing the neckband), and may transmit the detected acceleration and/or rotation as the sensor data. The processor may determine based on the sensor data whether the user is walking, standing, sitting or lying. The processor may also determine an incident of the user, e.g., if the user falls (such that the user may be injured and/or may need help getting up again) or if the user does not move for a long (e.g., exceeding a predetermined threshold) time (which may mean that the user is unconscious), and may notify a predefined contact according to the determined incident.

Thus, the IMU may be used for observing general movement activities of the user as well as for fall detection.

In some embodiments, the sensor includes a wireless signal strength detection unit; and the determining of the state includes determining a position of the user based on a wireless signal strength detected by the wireless signal strength detection unit.

The wireless signal strength may include a received power of a wireless signal and may be measured, e.g., in decibel-milliwatt (dBm). The wireless signal may include a Wi-Fi (IEEE 802.11) signal (e.g., a beacon frame from an access point), a ZigBee signal, a Bluetooth signal, an ultra-wide band (UWB) signal, an NR signal, an LTE signal, a Global System for Mobile Communications (GSM) signal or the like. The wireless signal strength detection unit may be included in a wireless interface of the communication unit.

The processor may determine a position of the neckband (and, thus, of the user who is wearing the neckband) relative to an emitter of the wireless signal. For example, the wireless signal strength detection unit may determine a distance of the neckband from the emitter. For example, the wireless signal strength detection unit may detect a wireless signal strength of wireless signals from several emitters of wireless signals (e.g., from several Wi-Fi access points) and determine the position of the neckband based on triangulation between the emitters.

The storage unit may store position information of the emitter(s) of the wireless signal(s). The position information may indicate a corresponding position. For example, the processor may receive the position information for an emitter of a wireless signal from a database, e.g., via the internet. For example, the processor may receive the position information for an emitter of a wireless signal from an input by the user. For example, the processor may determine the position information for an emitter of a wireless signal based on simultaneous localization and mapping (SLAM). The storage unit may store an association of a position with a physical site (e.g., a room in an apartment or house). The association may be inserted by the user.

The position may correspond to an indoor position, e.g., a room in an apartment or house in which the user lives. However, the disclosure is not limited to indoor positions, and the position may as well correspond to an address and/or a position specified in a geographic coordinate system (GCS) or the like.

The processor may observe a movement of the user who is wearing the neckband around a house, based on Wi-Fi signal strength changes in a Wi-Fi signal from a router (e.g., wireless access point) and, in some embodiments, combined with an IMU measurement and other sensors. Only high-level statistics of the position of the user may be provided to a care person of the user in order to respect the privacy of the wearer.

In some embodiments, the state includes a predefined position pattern of the user; and the action includes reporting an indication of the predefined position pattern.

The predefined position pattern may correspond to a time series of determined positions of the user and may indicate how and where the user has moved.

For example, the predefined position pattern may indicate that the user has moved out of a predefined house or apartment. If the user is demented, he might get lost and/or might get involved in an accident. Therefore, the processor may notify a predefined contact that the user has moved out of the predefined house our apartment.

For example, the predefined position pattern may indicate that the user has not moved for some time. A reason for not moving might be that the user is unconscious. Thus, the processor may notify a predefined contact to look after the user.

In some embodiments, the state includes entering a predefined room; and the action includes controlling lights of the room to be switched on.

For example, if the user does not switch on the light, he might stumble or fall and get hurt in a dark room where a light is turned off. To prevent such an accident, the processor may control a light (e.g., a smart light) in the room (e.g., via Wi-Fi, ZigBee or the like) to be switched on when the user enters the room. The processor may determine that the light is turned off via communication with a smart light in the room (e.g., via ZigBee or Wi-Fi) and/or may measure a brightness based on a brightness sensing unit included in the sensor.

In some embodiments, the sensor includes a microphone; the state includes a call for help; and the action includes requesting help from a predefined contact.

For example, the processor may identify a scream or call for help by the user and may first notify a predefined contact (e.g., a care person) and then, if needed, may call emergency services.

In some embodiments, the state includes a vital sign of the user.

The vital sign may include biological information that may indicate a medical state of the user.

In some embodiments, the state includes an anomaly in the vital sign.

The anomaly may correspond to a medical emergency that requires quick response. The anomaly may indicate a disease of the user that should be examined further.

In some embodiments, the action includes triggering an emergency call based on the anomaly in the vital sign.

For example, if the anomaly corresponds to a medical emergency that requires quick response, the processor may notify a predefined contact that is configured as an emergency contact (e.g., a doctor, a nurse and/or an EMS), such that the user may receive quick medical treatment.

In some embodiments, the sensor includes an audio sensor; and the processor is configured to determine the vital sign based on the audio sensor.

The audio sensor may include a microphone. For example, the microphone may capture a breathing sound of the user.

In some embodiments, the determining of the vital sign is based on targeted audio beam forming.

Targeted audio beam forming may allow compensating for noise from an environment such that even gentle sounds from the user may be detected.

For example, the processor may observe, based on audio data acquired by the audio sensor based on targeted audio beam forming, a breathing rate of the user who is wearing the neckband, and may detect in the audio data an anomaly, e.g., a panic attack of the user. The processor may then notify a predefined contact to look after the user.

In some embodiments, the sensor includes an ultra-wide band (UWB) receiver; and the processor is configured to determine the vital sign based on a UWB signal received by the UWB receiver.

The UWB signal may have a bandwidth (e.g., a frequency interval in which a power spectral density is at least -10 dB) that exceeds a lesser of 500 Megahertz (MHz) and 20 % of an arithmetic center frequency of the UWB signal. For example, the neckband may include a UWB emitter that emits a predefined UWB signal, and the UWB signal received by the UWB may correspond to a reflection of the emitted predefined UWB signal from a scene. Based on the received UWB signal, the UWB receiver may detect a micromovement (e.g., breathing, heartbeat) of the user who is wearing the neckband. Based on the micromovement, the processor may determine a vital sign (e.g., breathing rate, pulse rate) of the user, and/or may determine that the user is wearing the neckband.

In some embodiments, the sensor includes an event-based vision sensor (EVS); and the processor is configured to determine the vital sign based on events detected by the EVS.

The EVS may be configured as a neuromorphic camera. The EVS may detect, with each pixel in a pixel array of the EVS, a brightness change in a portion of a scene imaged by the respective pixel. Each brightness change detected by a pixel may correspond to an event detected by the EVS. When a pixel of the EVS detects a brightness change, the pixel may fire (e.g., generate an event) independently from the other pixels of the pixel array. The EVS may detect a million events per second (without limiting the disclosure to this value).

The EVS may detect micromovements (e.g., breathing, heartbeat) of the user. The processor may determine the vital sign (e.g., breathing rate, pulse rate) based on the events detected by the EVS and/or may determine that the user is wearing the neckband based on the events detected by the EVS.

In some embodiments, the sensor includes a green light sensor; and the processor is configured to determine the vital sign based on a sensing of the green light sensor.

The neckband may include an illumination unit that emits green light (e.g., light with a wavelength of 530 nanometers, without limiting the disclosure to this value), and the green light sensor may measure an amount of green light reflected from a scene (e.g., from the user who is wearing the neckband). The amount of reflected green light may correspond to an absorption of green light by hemoglobin in a blood vessel of the user.

The absorption of green light by hemoglobin may depend on whether the hemoglobin is oxygenated, such that an oxygen saturation of the blood of the user may be determined based on the amount of reflected green light received by the green light sensor.

With each heartbeat of the user, an increased amount of blood (and, thus, of hemoglobin) may flow through a blood vessel of the user in a time interval that corresponds to a contraction of a heart of the user. Due to the increased amount of hemoglobin in the blood vessel, an absorption of green light of the blood vessel (and of a skin that includes the blood vessel) may change in accordance with the heartbeat. Thus, the processor may determine the pulse rate of the user based on the amount of reflected green light received by the green light sensor.

In some embodiments, the vital sign includes a breathing rate of the user.

The processor may detect, based on the breathing rate of the user, an anomaly of the breathing rate, such as an increased or decreased breathing rate, irregular breathing, or no breath at all. Based on the detected anomaly of the breathing rate, the processor may detect a panic attack, dyspnea, apnea, hyperventilation or the like of the user. The processor may perform an action (e.g., based on a type of the detected anomaly) such as logging and/or reporting the anomaly, notifying a predefined contact and/or triggering an emergency call.

In some embodiments, the vital sign includes a sleeping pattern of the user.

The processor may determine the sleeping pattern based on a determined breathing rate of the user. For example, the user may wear the neckband while the user is sleeping, or the user may put the neckband in a stand at a bedside of the user. The processor may log and/or report the sleeping pattern.

For example, the processor may perform an audio observation of sleeping patterns and a breathing rate overnight when the neckband is placed at a bedside of the user.

In some embodiments, the vital sign includes a pulse rate of the user.

The pulse rate may indicate a heartbeat of the user and may correspond to a heartbeat frequency of the user. The processor may determine, based on the pulse rate, an irregularity of the heartbeat frequency and/or a cardiac arrest. The processor may perform an action such as logging and/or reporting the pulse rate, notifying a predefined contact and/or triggering an emergency call according to the determined pulse rate.

In some embodiments, the processor is further configured to connect to hearing aids worn by the user for providing audio output through the hearing aids.

The processor may generate an audio signal and transmit the audio signal to the hearing aids such that the hearing aids provide to the user audio output that corresponds to the audio signal. For example, the processor may control the communication unit to transmit the audio signal via an audio induction loop, and the audio signal may be received by a telecoil (T-coil) in the hearing aids. For example, the processor may control the communication unit to transmit the audio signal via Wi-Fi, Bluetooth, Bluetooth Low Energy (LE) Audio, via Infrared or the like to the hearing aids.

In some embodiments, the neckband further includes a loudspeaker; and the processor is further configured to output an audio signal via the loudspeaker for locating the neckband.

For example, if the user has put down the neckband and does not remember where he has put the neckband, the user may remotely (e.g., via a smartphone) activate a "find me" functionality of the neckband. When performing the "find me" functionality, the processor may output the audio signal (e.g., a predefined melody, a beep, a tone, a recorded speech or the like) such that the user may easier find the device by following the output audio signal.

In some embodiments, the sensor includes a microphone; and the processor is further configured to detect a mood of the user based on audio input from the user captured by the microphone.

The mood may include joy, sadness, fear, boredom or the like. For example, the processor may detect the mood based on words spoken by the user and/or based on a speech melody of the user.

For example, the processor may perform general mood identification based on audio inputs.

The processor may log the detected mood and/or notify a predefined contact of the detected mood. For example, when the processor detects a negative mood such as sadness, fear or boredom, the processor may notify the predefined contact to look after the user.

In some embodiments, the neckband includes one or more large tactile buttons for easy control of the neckband. The button(s) may be provided at a casing of the neckband and may be large enough for being operated by an elderly user.

In some embodiments, the neckband includes functionality for providing a positive experience to the user who is wearing the neckband.

The functionality may include connecting to a television (TV), radio, computer, (smart)phone or the like for providing audio/music to the user.

The functionality may include performing and/or accepting phone calls to contacts such as relatives, doctors, etc. For example, the neckband may provide a button for initiating a phone call to a predefined contact. For example, the neckband may include a microphone for capturing speech of the user during the phone call and a loudspeaker for outputting a speech received during the phone call.

The functionality may include connecting to hearing aids if the user has one. For example, an audio signal received or generated by the processor may be transmitted to the hearing aids instead of being output through a loudspeaker of the neckband.

The functionality may include providing an audio indication of a phone call, of a door bell, and/or of any smart devices (e.g., kitchen appliance indications (given the compatibility)).

The functionality may include any other fully customizable reminders. For example, the processor may output an audio signal for reminding the user to take predefined medicine at predefined times, to drink enough, to send birthday greetings to a predefined friend/relative and/or to get ready for a planned consultation with a doctor.

The functionality may include artificial intelligence (AI) based audio assistant functions such as providing entertainment (e.g., jokes, reading books, stories, audio-based games), providing a summary of the day, weather, news, any alerts and/or calendar event notifications, managing a shopping list (e.g., both record the shopping list and read it out later in a shop), and/or providing a public transport schedule and navigation when walking/driving (e.g., based on a connection to a smartphone).

In some embodiments, the neckband is compatible with audio-based services and voice-activated devices, which may provide good user experience and will to wear the neckband. For example, the neckband may be directly connected to and may utilize a functionality of voice-activated devices, such as Google Home, Amazon Alexa, Siri, or the like. Additionally, the neckband may provide an emergency calling functionality and/or a pre-set contact calling functionality at a press of a button such that no voice commands may be needed for calling.

In some embodiments, the neckband may be configured as an audio neckband for personal care.

In some instances, known wearable devices are not tailored for assisted living and are in wristband or similar format. The neckband according to the present technology, however, may be easy to put on and take off due to the form factor of a neckband, and may additionally provide localization information. Within an apartment of a user who is wearing the neckband, localization may be based on observing a strength of a wireless signal (Wi-Fi, Bluetooth, ZigBee, etc.) and/or of an ultra-wide band (UWB) signal. Thus, the localization functionality of the neckband may be fully privacy aware. The neckband may also provide additional assisted living functionality such as fall detection, emergency calling etc.

The neckband may locally detect a state of the user who is wearing the neckband from measured physiological data of the user and may send state information that indicates the detected state to a person/entity who is authorized to receive the information (e.g., to a predefined contact). The neckband may observe the state of the user both when the user is wearing the device as well as when the neckband is charging overnight, e.g., if the neckband is placed by the bedside close to the user while asleep.

Some embodiments pertain to a method for controlling the neckband as described above, wherein the method includes: acquiring sensor data from the sensor of the neckband; determining, based on the sensor data, a state of a user who is wearing the neckband on his shoulder; and performing an action according to the determined state.

For example, the method may be performed by the neckband of any embodiment described above. The method may be configured according to a functionality of the neckband, and any feature described with reference to the neckband may correspond to an according feature of the method.

The method as described herein is also implemented in some embodiments as a computer program causing a computer and/or a processor to perform the method, when being carried out on the computer and/or processor. In some embodiments, also a non-transitory computer-readable recording medium is provided that stores therein a computer program product, which, when executed by a processor, such as the processor described above, causes the method described herein to be performed.

Returning to Fig. 1, Fig. 1 illustrates a neckband 1 according to an embodiment.

A of Fig. 1 provides a detailed view of the neckband 1. The neckband 1 includes a right portion 2, a left portion 3 and a middle portion 4. The middle portion 4 is flexible and connects the right portion 2 and the left portion 3.

Each of the right portion 2 and the left portion 3 includes a loudspeaker 5a and 5b, respectively. The loudspeakers 5a and 5b are arranged such that they are positioned below a right or left ear, respectively, of a user who is wearing the neckband 1, such that sound output by the loudspeakers 5a and 5b can be directed at ears of the user.

Each of the right portion 2 and the left portion 3 further includes a microphone 6a and 6b, respectively. The microphones 6a and 6b are arranged at a front side of the right and left portions 2 and 3, respectively, such that speech of the user who is wearing the neckband 1 can be captured by microphones 6a and 6b based on targeted audio beam forming targeted at a mouth of the user.

The neckband 1 also includes buttons 7a, 7b, 7c and 7d arranged on both the right portion 2 and the left portion 3. The buttons 7a to 7d are large and tactile for easy control of the neckband 1 even by an elderly user.

B of Fig. 1 illustrates a user wearing the neckband 1. The right portion 2 lies on a right shoulder of the user, the left portion 3 lies on a left shoulder of the user, and the middle portion 4 (not visible in B of Fig. 1) is provided behind a neck 8 of the user.

Thus, the neckband 1 lies convenient on the shoulders of the user and is easy to take on and to put off.

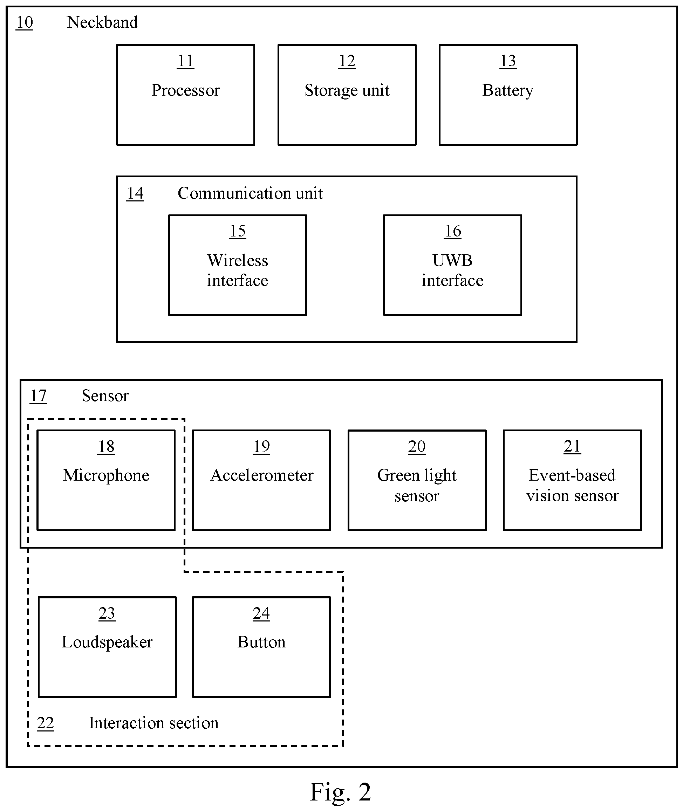

Fig. 2 illustrates a hardware configuration of a neckband 10 according to an embodiment. The neckband 10 is an example of the neckband 1 of Fig. 1. The neckband 10 includes a processor 11, a storage unit 12 and a battery 13.

The processor 11 is configured to control a function of the neckband 10 based on software instructions. The storage unit 12 stores, in a non-volatile part, the software instructions for the processor 11 and, in a volatile part, temporary data that have been generated by the processor 11 and/or that should be processed by the processor 11. The battery 13 stores electrical energy and provides the electrical energy for an operation of the neckband 10. A charge storing capacity of the battery 13 is large enough for allowing several days of operation of the neckband 10 without charging the battery 13 in between.

The neckband 10 further includes a communication unit 14 for peripheral devices and for connecting to the internet. The communication unit 14 further provides a localization functionality that allows locating the neckband 10 based on a wireless signal. The communication unit 14 includes a wireless interface 15 and an ultra-wide band (UWB) interface.

The wireless interface 15 is capable of performing wireless communication based on Wi-Fi, Bluetooth and ZigBee. The wireless interface 15 performs communication with smart devices, data sharing online communication and calls.

The UWB interface 16 is capable of performing localization of the neckband 10, automatic pairing with another device, and location-based device toggling (e.g., for switching on/off smart lights).

The neckband 10 further includes a sensor 17. The sensor 17 is configured to acquire sensor data and provide the acquired sensor data to the processor 11. The sensor 17 allows an observation of a user who is wearing the neckband 10 and tracking the user based on the acquired sensor data. The sensor 17 includes a microphone 18, an accelerometer 19, a green light sensor 20 and an event-based vision sensor (EVS) 21.

The microphone 18 is capable of capturing a speech and a breathing sound of the user who is wearing the neckband 10 as well as environmental sounds from a surrounding of the neckband 10. The microphone 18 is used for calls, voice commands, respiration monitoring and emergency identification. The microphones 6a and 6b of Fig. 1 are examples of the microphone 18.

The accelerometer 19 includes an inertial measurement unit (IMU) and is capable of detecting an acceleration and a rotation of the neckband 10. The accelerometer 19 is used for incident detection (including detecting a fall of the user who is wearing the neckband 10), standby mode (i.e., detecting that the neckband 10 has not been moved for a predefined amount of time and should therefore enter the standby mode, as well as detecting that the neckband 10 is moved again and should therefore wake up from the standby mode), reminders to wear the neckband 10 (if the user has put off the neckband 10), movement statistic collection, and a step counter (for counting how many steps the user has walked).

The green light sensor 20 emits green light with a wavelength of 530 nanometers (without limiting the disclosure thereto) and measures an amount of green light reflected back to the green light sensor 20. The green light sensor 20 is provided with direct contact to a skin of the user who is wearing the neckband 10 and, thus, is capable of measuring an absorption of green light by blood in the skin of the user. The green light sensor 20 is used for determining a pulse (rate) of the user as well as an oxygen saturation of blood of the user.

The EVS 21 is capable of detecting micromovements and is directed at a neck of the user who is wearing the neckband 10. The EVS 21 captures events of the neck and provides the captured events to the processor 11. The processor 11 determines, based on the events, micromovements of a carotid artery of the user and, based on these micromovements, a pulse rate of the user.

The processor 11 is configured to determine, based on the sensor data acquired by the sensor 17, a state of the user who is wearing the neckband 10 on his shoulders and perform an action according to the determined state.

The neckband 10 further includes an interaction section 22 that allows an interaction between the user and the neckband 10 and provides an entertainment functionality of the neckband 10. The interaction section 22 includes the microphone 18, a loudspeaker 23 and a button 24.

The loudspeaker 23 is capable of outputting a sound to the user and is used for outputting entertainment, music, calls, sound from (peripheral) devices, reminders and warnings. The loudspeakers 5a and 5b of Fig. 1 are examples of the loudspeaker 23.

The button 24 is capable of detecting when the user presses the button 24. When the user presses the button 24, the button 24 provides a corresponding signal to the processor 11, and the processor 11 performs a predefined action that is associated with the button 24. The action associated with the button 24 includes powering on/off the neckband 10, calling, and custom-programmed functionality. The buttons 7a to 7d of Fig. 1 are examples of the button 24 and include a pre-set contact calling button, an emergency calling button, an on/off button, and a custom programmable button.

Note that, in some embodiments, the neckband 10 does not include all units shown in Fig. 2 and/or a unit of the neckband 10 does not perform all functions described above. For example, the wireless interface 15 may not be capable of communicating via ZigBee. For example, the communication unit 14 may not include the UWB interface 16. For example, the sensor 17 may not include the accelerometer 19. For example, the sensor 17 may not include the green light sensor 20. For example, the sensor 17 may not include the EVS 21. For example, the processor 11 may not determine an oxygen saturation based on the green light sensor 20. The skilled person may find further variations of the neckband 10.

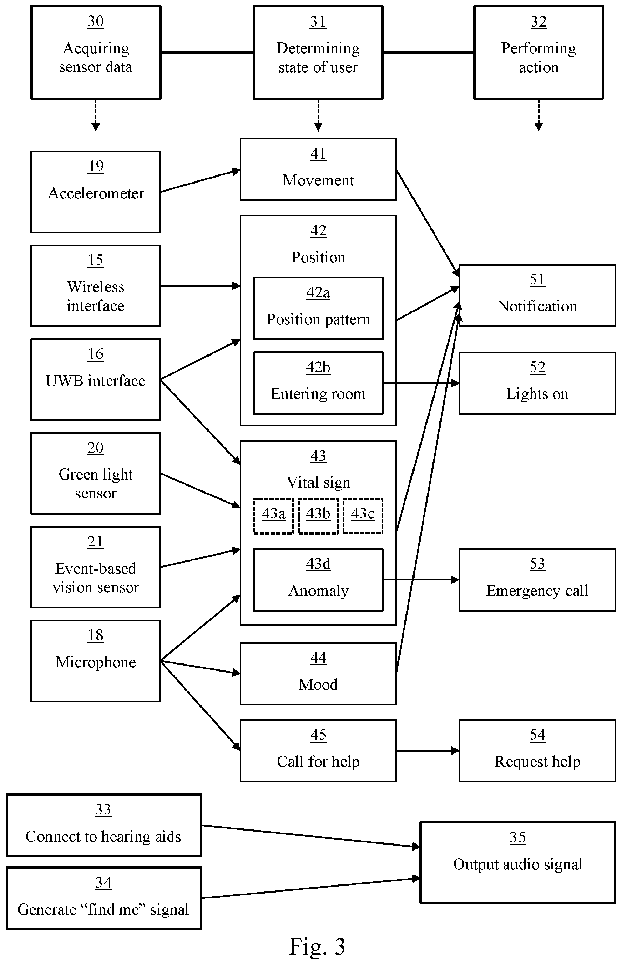

Fig. 3 illustrates a method according to an embodiment. The method controls a neckband (such as the neckband 1 of Fig. 1 and the neckband 10 of Fig. 2) and is an example of a method performed by the processor 11 of the neckband 10.

At 30, the processor 11 acquires sensor data from a sensor included in a neckband (e.g., the sensor 17 of the neckband 10). At 31, the processor 11 determines, based on the acquired sensor data, a state of a user who is wearing the neckband on his shoulder, as illustrated in B of Fig. 1. At 32, the processor 11 performs an action according to the determined state of the user. The action performed at 32 includes notifying 51 a predefined contact of the state of the user that has been determined at 31.

The acquiring of sensor data at 30 includes acquiring sensor data from the accelerometer 19 of Fig. 2, which includes an IMU. The processor 11 acquires, from the IMU, sensor data that indicate an acceleration and a rotation of the neckband 10 (and, thus, of the user who is wearing the neckband 10). The determining at 31 includes determining a movement 41 of the user based on the sensor data from the IMU. The movement 41 of the user is an example of a state of the user. The action performed at 32 includes notifying 51 the predefined contact of the movement 41.

The acquiring of sensor data at 30 includes acquiring sensor data from a wireless signal strength detection unit. Examples of a wireless signal strength detection unit are the wireless interface 15 and the UWB interface 16 of Fig. 2. The sensor data from the wireless signal strength detection unit indicate a received power (which is an example of a wireless signal strength) of a wireless signal detected by the wireless signal strength detection unit. The processor 11 determines, based on the sensor data (and, thus, on the detected wireless signal strength) from the wireless signal strength detection unit, a position 42 of the user. The position 42 is an example of a state of the user, and the notification 51 includes notifying the predefined contact of the determined position 42 of the user.

The processor 11 further determines, based on the position 42, a predefined position pattern 42a of the user. The position pattern 42a corresponds to a time series of determined positions 42 of the user and is a further example of a state of the user. The notification 51 includes reporting an indication of the position pattern 42a of the user.

The processor 11 further determines, based on the position 42, that the user is entering a predefined room. The entering 42b of the predefined room is an example of a state of the user. When the processor 11 determines that the user is entering 42b the predefined room, the processor 11 controls 52 lights of the room to be switched on, which is an example of the action performed at 32.

The state of the user, which the processor 11 determines at 31, includes a vital sign 43 of the user, and the notification 51 includes notifying the predefined contact of the determined vital sign 43. The vital sign 43 includes a breathing rate 43a of the user, a sleeping pattern 43b of the user and a pulse rate 43c of the user.

The acquiring of sensor data at 30 includes acquiring sensor data from an UWB receiver of the UWB interface 16 of Fig. 2. The sensor data from the UWB receiver (and, thus, from the UWB interface 16) represent a UWB signal received by the UWB receiver. The UWB signal indicates a micromovement of the user. The processor 11 determines at 31 the vital sign 43 based on the sensor data from the UWB interface 16.

The acquiring of sensor data at 30 includes acquiring sensor data from the green light sensor 20 of Fig. 2. The processor 11 determines the vital sign 43 at 31 based on the sensor data from the green light sensor 20 and, thus, based on a sensing of the green light sensor 20.

The acquiring of sensor data at 30 includes acquiring sensor data from the EVS 21 of Fig. 2. The sensor data from the EVS 21 indicate events detected by the EVS 21. The processor 11 determines the vital sign 43 at 31 based the sensor data from the EVS 21 and, thus, based on the events detected by the EVS 21.

The acquiring of sensor data at 30 includes acquiring sensor data from the microphone 18 of Fig. 2. The microphone 18 is an example of an audio sensor. The microphone 18 captures a sound from the user based on targeted audio beam forming, and the sensor data from the microphone 18 indicate the sound from the user captured based on targeted audio beam forming. The processor 11 determines the vital sign 43 at 31 based on the sensor data from the microphone 18 and, thus, based on the microphone 18 as well as on targeted audio beam forming.

The state of the user also includes an anomaly 43d in the vital sign 43. At 31, the processor 11 detects the anomaly 43d and triggers 53 an emergency call based on the anomaly 43d in the vital sign 43. The triggering 53 of the emergency call is an example of an action performed at 32.

As mentioned above, the processor 11 acquires, at 30, sensor data from the microphone 18. The microphone 18 captures audio input from the user, and the sensor data from the microphone 18 indicate the captured audio input from the user. The processor 11 detects, at 31, a mood 44 of the user based on the sensor data and, thus, based on the audio input from the user captured by the microphone 18. The notification 51 at 32 includes notifying the predefined contact of the detected mood 44.

The processor 11 also detects, based on the sensor data from the microphone 18, a call 45 for help by the user. The call 45 for help is an example of a state of the user. Based on the call 45 for help, the processor 11 requests 54 help from a predefined contact. The requesting 54 of help is an example of an action performed by the processor 11 at 32.

The method further includes connecting 33 to hearing aids worn by the user. The processor 11 connects at 33, via the communication unit 14, to the hearing aids for providing audio output through the hearing aids.

The method further includes generating, at 34, a "find me" signal. The "find me" signal is an audio signal that helps the user to find the neckband 10 if the user has put off the neckband 10 and does not remember where he has put the neckband 10. The user causes the processor 11 to generate the "find me" signal at 34 with a smartphone.

The method further includes outputting an audio signal at 35. The audio signal includes the "find me" signal generated at 34. The processor 11 controls the loudspeaker 23 of Fig. 2 to output the audio signal.

The audio signal also represents further content such as music played back by the processor 11, speech received by the processor 11 in a phone call as well as sound generated by an artificial intelligence (AI) audio assistant function of the processor 11. The processor 11 controls the loudspeaker 23 to output the further content at 35. If the neckband 11 has been connected to hearing aids of the user at 33, the further content is output via the hearing aids. However, even if the neckband has been connected to hearing aids at 33, the "find me" signal is output via the loudspeaker 23 of the neckband 10 (and not via the hearing aids) for locating the neckband 10.

It is noted that, in some embodiments, not all aspects of the method that have been described herein are performed.

For example, the processor 11 may not acquire sensor data from the accelerometer 19, or the processor 11 may not notify the movement 41 at 51 but, e.g., may only determine whether to enter or wake up from a standby mode based on the movement 41.

For example, the processor 11 may not receive sensor data from the wireless interface 15 and/or from the UWB interface 16, the processor 11 may not determine the position pattern 42a and/or the entering 42b of a room, the processor 11 may not notify the position 42 and/or the position pattern 42a at 51, and/or the processor 11 may not control the lights to be switched on at 52.

For example, the processor 11 may determine the vital sign 43 based on only one, two or three of the UWB interface 16, the green light sensor 20, the EVS 21 and the microphone 18. The processor 11 may also determine the vital sign 43 based on another sensor not mentioned above. The vital sign 43 determined by the processor 11 may include only one or two of the breathing rate 43a, the sleeping pattern 43b and the pulse rate 43c of the user. The processor 11 may also determine further vital signs of the user, e.g., an oxygen saturation and/or a blood sugar level of the user. The processor 11 may not notify, at 51, the predefined contact of the vital sign 43 but only log the vital sign 43 and/or monitor the vital sign 43 for triggering 53 an emergency call. The processor 11 may not be configured to trigger 53 an emergency call but only to notify 51 and/or log the vital sign 43.

For example, the processor 11 may not detect a mood 44 of the user and/or may not detect a call 45 for help.

For example, the processor 11 may not be configured to connect 33 to hearing aids and/or generate 34 a "find me" signal.

Thus, some embodiments only pertain to some, but not all aspects of the method of Fig. 3. The skilled person may find a suitable combination of aspects of the method.

Fig. 4 illustrates an embodiment of a general-purpose computer 150. The computer 150 can be implemented such that it can basically function as any type of information processing apparatus, for example, the neckband 1 of Fig. 1 or the neckband 10 of Fig. 2. The computer 150 has components 151 to 161, which can form a circuitry, such as a circuitry of the neckband 10 of Fig. 2 (e.g., the processor 11, the storage unit 12 and/or the communication unit 14), as described herein.

Embodiments which use software, firmware, programs or the like for performing the methods as described herein can be installed on computer 150, which is then configured to be suitable for the concrete embodiment.

The computer 150 has a CPU 151 (Central Processing Unit), which can execute various types of procedures and methods as described herein, for example, in accordance with programs stored in a read-only memory (ROM) 152, stored in a storage 157 and loaded into a random-access memory (RAM) 153, stored on a medium 160 which can be inserted in a respective drive 159, etc.

The CPU 151, the ROM 152 and the RAM 153 are connected with a bus 161, which in turn is connected to an input/output interface 154. The number of CPUs, memories and storages is only exemplary, and the skilled person will appreciate that the computer 150 can be adapted and configured accordingly for meeting specific requirements which arise, when it functions as a base station or as user equipment (end terminal).

At the input/output interface 154, several components are connected: an input 155, an output 156, the storage 157, a communication interface 158 and the drive 159, into which a medium 160 (compact disc, digital video disc, compact flash memory, or the like) can be inserted.

The input 155 can be a pointer device (mouse, graphic table, or the like), a keyboard, a microphone, a camera, a touchscreen, an eye-tracking unit etc.

The output 156 can have a display (liquid crystal display, cathode ray tube display, light emittance diode display, etc.; e.g., included in a touchscreen), loudspeakers, etc.

The storage 157 can have a hard disk, a solid-state drive, a flash drive and the like.

The communication interface 158 can be adapted to communicate, for example, via a local area network (LAN), wireless local area network (WLAN), mobile telecommunications system (GSM, UMTS, LTE, NR etc.), Bluetooth, near-field communication (NFC), infrared, etc.

It should be noted that the description above only pertains to an example configuration of computer 150. Alternative configurations may be implemented with additional or other sensors, storage devices, interfaces or the like. For example, the communication interface 158 may support other radio access technologies than the mentioned UMTS, LTE and NR.

It should be recognized that the embodiments describe methods with an exemplary ordering of method steps. The specific ordering of method steps is however given for illustrative purposes only and should not be construed as binding. For example, the ordering of 41, 42, 43, 44 and/or 45 in the embodiment of Fig. 3 may be exchanged. Also, the ordering of 51, 52, 53 and/or 54 in the embodiment of Fig. 3 may be exchanged. Further, also, the ordering of 33 and 34 in the embodiment of Fig. 3 may be exchanged. Other changes of the ordering of method steps may be apparent to the skilled person.

Please note that the division of the neckband 10 into units 11 to 24 is only made for illustration purposes and that the present disclosure is not limited to any specific division of functions in specific units. For instance, the neckband 10 and/or the processor 11 could be implemented by a respective programmed processor, field programmable gate array (FPGA) and the like.

The method can also be implemented as a computer program causing a computer and/or a processor, such as processor 11 discussed above, to perform the method, when being carried out on the computer and/or processor. In some embodiments, also a non-transitory computer-readable recording medium is provided that stores therein a computer program product, which, when executed by a processor, such as the processor described above, causes the method described to be performed.

All units and entities described in this specification and claimed in the appended claims can, if not stated otherwise, be implemented as integrated circuit logic, for example on a chip, and functionality provided by such units and entities can, if not stated otherwise, be implemented by software.

In so far as the embodiments of the disclosure described above are implemented, at least in part, using software-controlled data processing apparatus, it will be appreciated that a computer program providing such software control and a transmission, storage or other medium by which such a computer program is provided are envisaged as aspects of the present disclosure.

Note that the present technology can also be configured as described below.
(1) A neckband to be worn on a shoulder of a user, the neckband comprising:
   a sensor configured to acquire sensor data; and
   a processor configured to:
      determine, based on the sensor data, a state of a user who is wearing the neckband on his shoulder; and
      perform an action according to the determined state.
(2) The neckband of (1),
   wherein the action includes notifying a predefined contact of the determined state.
(3) The neckband of (1) or (2),
   wherein the sensor includes an inertial measurement unit; and
   wherein the determining of the state of the user includes determining a movement of the user based on the inertial measurement unit.
(4) The neckband of any one of (1) to (3),
   wherein the sensor includes a wireless signal strength detection unit; and
   wherein the determining of the state includes determining a position of the user based on a wireless signal strength detected by the wireless signal strength detection unit.
(5) The neckband of (4),
   wherein the state includes a predefined position pattern of the user; and
   wherein the action includes reporting an indication of the predefined position pattern.
(6) The neckband of (4) or (5),
   wherein the state includes entering a predefined room; and
   wherein the action includes controlling lights of the room to be switched on.
(7) The neckband of any one of (1) to (6),
   wherein the sensor includes a microphone;
   wherein the state includes a call for help; and
   wherein the action includes requesting help from a predefined contact.
(8) The neckband of any one of (1) to (7),
   wherein the state includes a vital sign of the user.
(9) The neckband of (8),
   wherein the state includes an anomaly in the vital sign.
(10) The neckband of (9),
   wherein the action includes triggering an emergency call based on the anomaly in the vital sign.
(11) The neckband of any one of (8) to (10),
   wherein the sensor includes an audio sensor; and
   wherein the processor is configured to determine the vital sign based on the audio sensor.
(12) The neckband of (11),
   wherein the determining of the vital sign is based on targeted audio beam forming.
(13) The neckband of any one of (8) to (12),
   wherein the sensor includes an ultra-wide band receiver; and
   wherein the processor is configured to determine the vital sign based on an ultra-wide band signal received by the ultra-wide band receiver.
(14) The neckband of any one of (8) to (13),
   wherein the sensor includes an event-based vision sensor; and
   wherein the processor is configured to determine the vital sign based on events detected by the event-based vision sensor.
(15) The neckband of any one of (8) to (14),
   wherein the sensor includes a green light sensor; and
   wherein the processor is configured to determine the vital sign based on a sensing of the green light sensor.
(16) The neckband of any one of (8) to (15),
   wherein the vital sign includes a breathing rate of the user.
(17) The neckband of any one of (8) to (16),
   wherein the vital sign includes a sleeping pattern of the user.
(18) The neckband of any one of (8) to (17),
   wherein the vital sign includes a pulse rate of the user.
(19) The neckband of any one of (1) to (18),
   wherein the processor is further configured to connect to hearing aids worn by the user for providing audio output through the hearing aids.
(20) The neckband of any one of (1) to (19),
   wherein the neckband further includes a loudspeaker; and
   wherein the processor is further configured to output an audio signal via the loudspeaker for locating the neckband.
(21) The neckband of any one of (1) to (20),
   wherein the sensor includes a microphone; and
   wherein the processor is further configured to detect a mood of the user based on audio input from the user captured by the microphone.
(22) Method for controlling a neckband in accordance with any one of (1) to (21), the method comprising:
   acquiring sensor data from the sensor of the neckband;
   determining, based on the sensor data, a state of a user who is wearing the neckband on his shoulder; and
   performing an action according to the determined state.
(23) The method of (22),
   wherein the action includes notifying a predefined contact of the determined state.
(24) The method of (22) or (23),
   wherein the sensor includes an inertial measurement unit; and
   wherein the determining of the state of the user includes determining a movement of the user based on the inertial measurement unit.
(25) The method of any one of (22) to (24),
   wherein the sensor includes a wireless signal strength detection unit; and
   wherein the determining of the state includes determining a position of the user based on a wireless signal strength detected by the wireless signal strength detection unit.
(26) The method of (25),
   wherein the state includes a predefined position pattern of the user; and
   wherein the action includes reporting an indication of the predefined position pattern.
(27) The method of (25) or (26),
   wherein the state includes entering a predefined room; and
   wherein the action includes controlling lights of the room to be switched on.
(28) The method of any one of (22) to (27),
   wherein the sensor includes a microphone;
   wherein the state includes a call for help; and
   wherein the action includes requesting help from a predefined contact.
(29) The method of any one of (22) to (28),
   wherein the state includes a vital sign of the user.
(30) The method of (29),
   wherein the state includes an anomaly in the vital sign.
(31) The method of (30),
   wherein the action includes triggering an emergency call based on the anomaly in the vital sign.
(32) The method of any one of (29) to (31),
   wherein the sensor includes an audio sensor; and
   wherein the method comprises determining the vital sign based on the audio sensor.
(33) The method of (32),
   wherein the determining of the vital sign is based on targeted audio beam forming.
(34) The method of any one of (29) to (33),
   wherein the sensor includes an ultra-wide band receiver; and
   wherein the method comprises determining the vital sign based on an ultra-wide band signal received by the ultra-wide band receiver.
(35) The method of any one of (29) to (34),
   wherein the sensor includes an event-based vision sensor; and
   wherein the method comprises determining the vital sign based on events detected by the event-based vision sensor.
(36) The method of any one of (29) to (35),
   wherein the sensor includes a green light sensor; and
   wherein the method comprises determining the vital sign based on a sensing of the green light sensor.
(37) The method of any one of (29) to (36),
   wherein the vital sign includes a breathing rate of the user.
(38) The method of any one of (29) to (37),
   wherein the vital sign includes a sleeping pattern of the user.
(39) The method of any one of (29) to (38),
   wherein the vital sign includes a pulse rate of the user.
(40) The method of any one of (22) to (39),
   wherein the method further comprises connecting to hearing aids worn by the user for providing audio output through the hearing aids.
(41) The method of any one of (22) to (40),
   wherein the neckband further includes a loudspeaker; and
   wherein the method further comprises outputting an audio signal via the loudspeaker for locating the neckband.
(42) The method of any one of (22) to (41),
   wherein the sensor includes a microphone; and
   wherein the method further comprises detecting a mood of the user based on audio input from the user captured by the microphone.
(43) A computer program comprising program code causing a computer to perform the method according to anyone of (22) to (42), when being carried out on a computer.
(44) A non-transitory computer-readable recording medium that stores therein a computer program product, which, when executed by a processor, causes the method according to anyone of (22) to (42) to be performed.

## Claims

1. A neckband to be worn on a shoulder of a user, the neckband comprising:
a sensor configured to acquire sensor data; and
a processor configured to:
determine, based on the sensor data, a state of a user who is wearing the neckband on his shoulder; and
perform an action according to the determined state.

2. The neckband of claim 1,
wherein the action includes notifying a predefined contact of the determined state.

3. The neckband of claim 1 or 2,
wherein the sensor includes an inertial measurement unit; and
wherein the determining of the state of the user includes determining a movement of the user based on the inertial measurement unit.

4. The neckband of any one of the preceding claims,
wherein the sensor includes a wireless signal strength detection unit; and
wherein the determining of the state includes determining a position of the user based on a wireless signal strength detected by the wireless signal strength detection unit.

5. The neckband of claim 4,
wherein the state includes a predefined position pattern of the user; and
wherein the action includes reporting an indication of the predefined position pattern.

6. The neckband of claim 4 or 5,
wherein the state includes entering a predefined room; and
wherein the action includes controlling lights of the room to be switched on.

7. The neckband of any one of the preceding claims,
wherein the sensor includes a microphone;
wherein the state includes a call for help; and
wherein the action includes requesting help from a predefined contact.

8. The neckband of any one of the preceding claims,
wherein the state includes a vital sign of the user.

9. The neckband of claim 8,
wherein the state includes an anomaly in the vital sign.

10. The neckband of claim 9,
wherein the action includes triggering an emergency call based on the anomaly in the vital sign.

11. The neckband of any one of claims 8 to 10,
wherein the sensor includes an audio sensor; and
wherein the processor is configured to determine the vital sign based on the audio sensor;
in particular wherein the determining of the vital sign is based on targeted audio beam forming.

12. The neckband of any one of claims 8 to 11,
wherein the sensor includes an ultra-wide band receiver, and
wherein the processor is configured to determine the vital sign based on an ultra-wide band signal received by the ultra-wide band receiver; and/or
wherein the sensor includes an event-based vision sensor, and
wherein the processor is configured to determine the vital sign based on events detected by the event-based vision sensor; and/or
wherein the sensor includes a green light sensor, and
wherein the processor is configured to determine the vital sign based on a sensing of the green light sensor.

13. The neckband of any one of claims 8 to 12,
wherein the vital sign includes a breathing rate of the user; and/or
wherein the vital sign includes a sleeping pattern of the user; and/or
wherein the vital sign includes a pulse rate of the user.

14. The neckband of any one of the preceding claims,
wherein the processor is further configured to connect to hearing aids worn by the user for providing audio output through the hearing aids.

15. The neckband of any one of the preceding claims,
wherein the neckband further includes a loudspeaker; and
wherein the processor is further configured to output an audio signal via the loudspeaker for locating the neckband.
